# EUROPEAN PATENT APPLICATION

(11) **EP 2 826 452 A1**
(43) Date of publication of application: **21.01.2015**
(21) Application number: 14177703.7
(22) Date of filing: 18.07.2014
(51) Int. Cl.: A61F 13/62, A61F 13/496, A61F 13/56, A61F 13/15

(54) **Apparatus and method for forming a pant-type diaper with refastenable side seams**

(30) Priority: 18.07.2013 US 201361847793 P
(71) Applicant: Curt G. Joa, Inc., Sheboygan Falls, Wisconsin 53085 (US)
(72) Inventor: McCabe,, John A, Sheboygan Falls, WI Wisconsin 53085 (US)
(74) Representative: Potter Clarkson LLP

(57) **Abstract**

A pants type disposable undergarment is provided which is equipped with a pre-fastened pull-on pant with a side lap seam formed by the methods of the present invention, and methods for producing such disposable undergarments.

## Description

### Background of the Invention

The present invention related to disposable undergarments and more particularly, a pants type undergarment which is equipped with refastenable side seams.

Disposable undergarments of the children's training pant type, or of the adult incontinence type, are generally made up of two nonwoven layers of material with elastic strands of material placed between the two nonwoven layers of material thus creating an elastic web laminate.

### Summary of the Invention

The present invention discloses methods of forming a pants type diaper with refastenable side seams. A pants type disposable undergarment is provided which is equipped with a pre-fastened pull-on pant with a side lap seam formed by the methods of the present invention. Top and bottom portions of a side panel assembly are folded over a stretch portion of the side panel, and both the top and bottom portions are bonded to the stretch portion of the side panel, at first temporary bond points and at second ultrasonic or mechanical bond portions. The second ultrasonic or mechanical bond portions are overlain with a hook-type fastener for later bonding with the first temporary bond points, to form a lap seam at the left and right sides of the waist of a wearer. A folded product is produced that is pre-fastened in this manner, but the bond between the hook-type fastener and the first temporary bonded portion can be released and re-fastened if desired.

A product and method to produce a resealable pant such that the front and rear waist side panel regions can be easily engaged with one another.

A method to produce a resealable pant in such a manner so that the front and rear waist side panels are a single piece during manufacturing is disclosed. A hinged panel with a resealable fastener to align with the mating side panel when folded to form a lap seam is also disclosed.

Several variations of manufacturing techniques and diaper products produced thereby are shown. For instance, an extension panel attached to outside surfaces of an elastic front and back side panels is shown. This extension panel (for instance, formed of a non-woven material) is coupled to at least one of a back or front panel. The hook portion may or may not be attached to the nonwoven extension panel, and the hook may face the body or away from the body. The nonwoven extension panels wind up on the outside of the product resulting in a refastenable lap seam.

### Brief Description of the Drawings

Fig. 1 is a perspective view of an undergarment produced according to the present invention.
Fig. 2 is a top planar view of a composite of material used to form a side panel.
Fig. 3 is a top planar view of a side panel assembly of embodiment shown in Fig. 2, with the top and bottom non-stretch materials folded over.
Fig. 4 is a top planar view of assembly of embodiment shown in Fig. 3, with the top and bottom non-stretch materials folded over and bonded in portions to the stretch portions.
Fig. 5 is a top planar view of assembly of embodiment shown in Fig. 4, with the top and bottom non-stretch materials folded over and bonded in portions to the stretch portions, and a hook material applied to the back side panel proximate to the bond area.
Fig. 6 is a top planar view of assembly of embodiment shown in Fig. 5, with the top and bottom non-stretch materials folded over and bonded in portions to the stretch portions, and a hook material applied to the back side panel proximate to the bond area, with portions of the assembly removed by die cut to facilitate shaping of the side panel.
Fig. 7 is a top planar view of assembly of embodiment shown in Fig. 6, with the top and bottom non-stretch materials folded over and bonded in portions to the stretch portions, and a hook material applied to the back side panel proximate to the bond area, with portions of the assembly removed by die cut to facilitate shaping of the side panel, and the side panel slit and spread apart.
Fig. 8 is a top planar view of assembly of embodiment shown in Fig. 7, with the top and bottom non-stretch materials folded over and bonded in portions to the stretch portions, and a hook material applied to the back side panel proximate to the bond area, with portions of the assembly removed by die cut to facilitate shaping of the side panel, and the side panel slit and spread apart, bonded to a top sheet material.
Fig. 9 is a top planar view of assembly of embodiment shown in Fig. 8, with the top and bottom non-stretch materials folded over and bonded in portions to the stretch portions, and a hook material applied to the back side panel proximate to the bond area, with portions of the assembly removed by die cut to facilitate shaping of the side panel, and the side panel slit and spread apart, bonded to a top sheet material, and this combination combined with a core and back sheet material.
Fig. 10 is a top planar view of assembly of embodiment shown in Fig. 9, with the top and bottom non-stretch materials folded over and bonded in portions to the stretch portions, and a hook material applied to the back side panel proximate to the bond area, with portions of the assembly removed by die cut to facilitate shaping of the side panel, and the side panel slit and spread apart, bonded to a top sheet material, and this combination combined with a core and back sheet material, showing the finished product prior to folding.
Fig. 11 is a finished product after folding, and prior to packaging.
Fig. 12 is an exploded cross sectional view of the pre-fastened pull-on pant with a side lap seam formed by the methods of the present invention.
Fig. 13 is schematic representation of formation of an alternate embodiment of the present invention, disclosed is a nonwoven tab process with a continuous hook formation, with the hooks away from the body.
Fig. 14 is a schematic representation of formation of an alternate embodiment of the present invention, disclosed is a nonwoven tab process with a discrete hook formation, with the hooks away from the body.
Fig. 15 is a schematic representation of formation of an alternate embodiment of the present invention, disclosed is a nonwoven tab process with a discrete hook formation extending into a die cut region, with the hooks away from the body.
Fig. 16 is a schematic representation of formation of an alternate embodiment of the present invention, disclosed is a nonwoven tab process with a discrete hook overlapping cut formation, with the hooks away from the body.
Fig. 17 is a schematic representation of formation of an alternate embodiment of the present invention, disclosed is a nonwoven tab process with a multiple discrete hook overlapping cut formation, with the hooks away from the body.
Fig. 18 is a schematic representation of formation of an alternate embodiment of the present invention, disclosed is a nonwoven tab process with hooks on the front side panel, with the hooks away from the body.
Fig. 19 is a schematic representation of formation of an alternate embodiment of the present invention, disclosed is a nonwoven tab process with hooks on the front side panel, and nonwoven tabs on the back panel, with hooks toward the body.
Fig. 20 is a schematic representation of formation of an alternate embodiment of the present invention, disclosed is a nonwoven tab process with hooks on the rear or back side panel, and nonwoven tabs on the front panel, with hooks toward the body.
Fig. 21 is a plan view of a product produced according to the present invention.
Figs. 22-24 are cross sectional views of a product produced according to the present invention.
Figs. 25- 28 are in-process, plan, and side views of a fourth alternate embodiment of a diaper of the present invention;
Figs. 29- 32 are in-process, plan, and side views of a fifth alternate embodiment of a diaper of the present invention;
Figs. 33- 36 are in-process, plan, and side views of a sixth alternate embodiment of a diaper of the present invention;
Figs. 37- 40 are in-process, plan, and side views of a seventh alternate embodiment of a diaper of the present invention;
Figs. 41- 44 are in-process, plan, and side views of an eighth alternate embodiment of a diaper of the present invention;
Figs. 45- 48 are in-process, plan, and side views of a ninth alternate embodiment of a diaper of the present invention;
Figs. 49- 52 are in-process, plan, and side views of a tenth alternate embodiment of a diaper of the present invention;
Figs. 53- 56 are in-process, plan, and side views of an eleventh alternate embodiment of a diaper of the present invention;
Figs. 57- 60 are in-process, plan, and side views of a twelfth alternate embodiment of a diaper of the present invention.
Figs. 61-63 are in-process, plan, and side views of a thirteenth alternate embodiment of a diaper of the present invention.
Figs. 64 is an in-process and side view of a fourteenth alternate embodiment of a diaper of the present invention.

### Description of the Preferred Embodiment

Although the disclosure hereof is detailed and exact to enable those skilled in the art to practice the invention, the physical embodiments herein disclosed merely exemplify the invention which may be embodied in other specific structures. While the preferred embodiment has been described, the details may be changed without departing from the invention, which is defined by the claims.

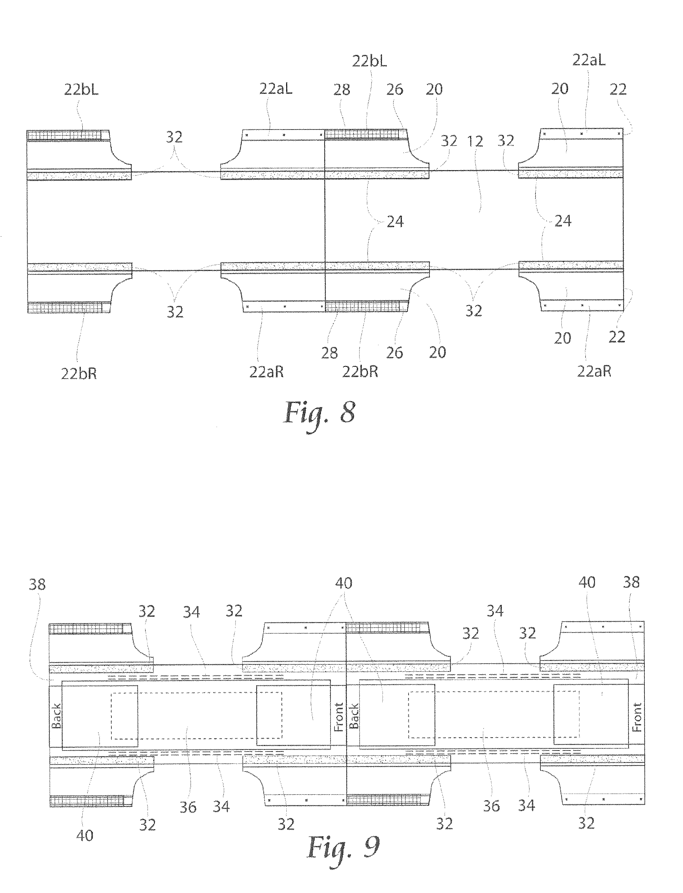

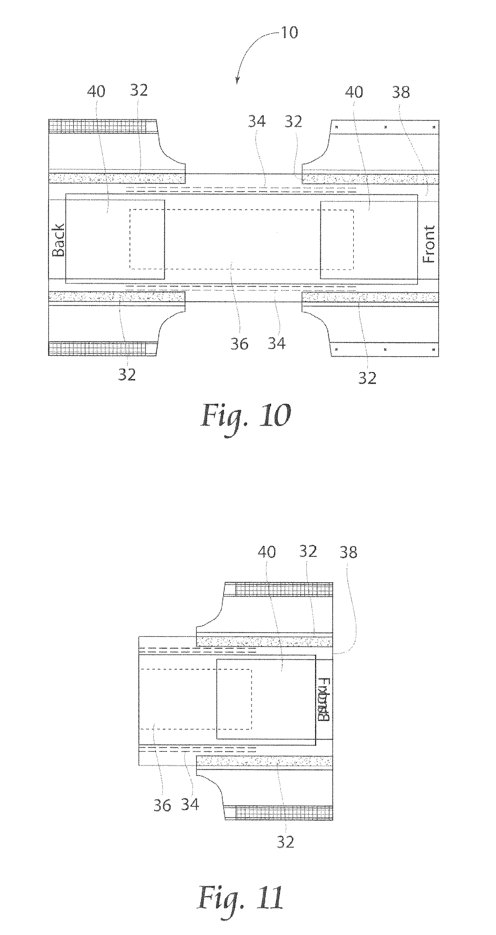

Referring to the Figures generally, two adjacent products are shown constructed next to one another because the products 10 are preferably constructed on a continuous processing system for later separation to form individual diaper products (see, e.g., Fig. 9 prior to separation to form individual products in Fig. 10).

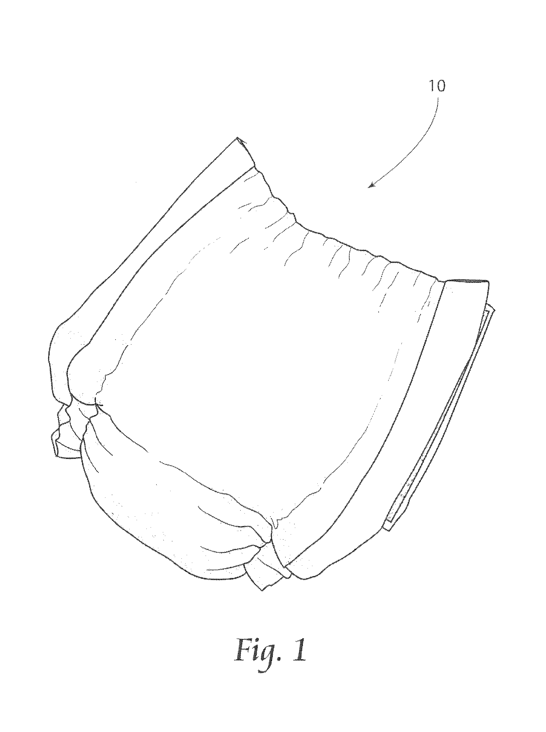

Fig. 1 is a perspective view of an undergarment 10 produced according to the present invention.

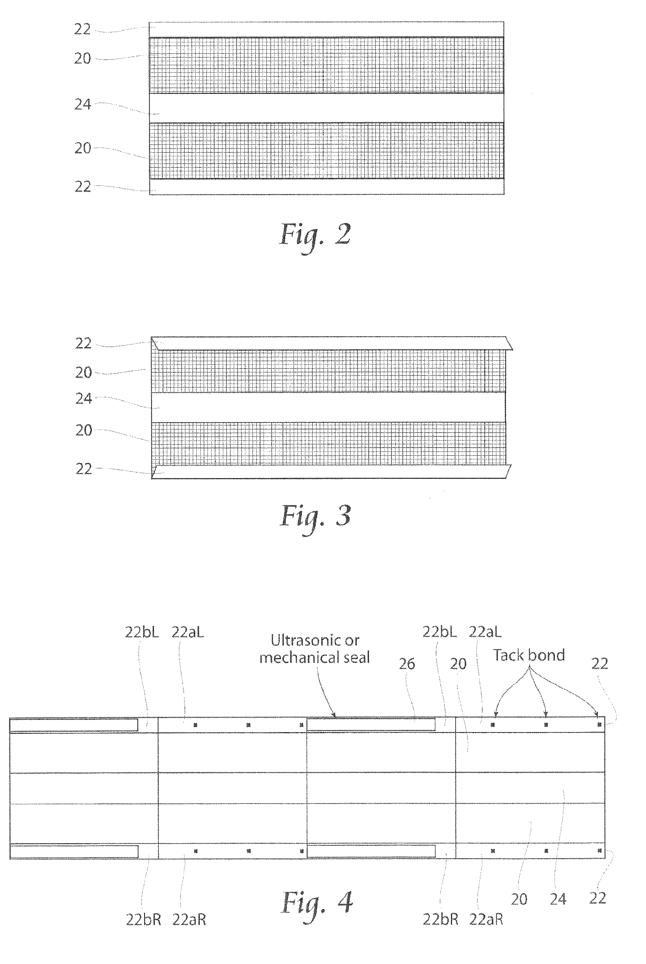

Referring now to Fig. 2 a top planar view of a composite of material used to form a side panel is shown. First and second segments of material 22 are provided at the top and bottom of the composite, along with first and second segments of preferably stretchy material 20. The first and second segments of material 22 can be formed of the same piece of stretch material 20, or formed separately of stretch or non-stretch material and then bonded to the stretch material 20. A segment of non-stretch material 24 is provided between the first and second segments 20 as shown.

Referring now to Fig. 3, the first and second segments of material 22 are folded either over or under the adjoining first and second segments of stretch material 20. This can be done with a folding machine known in the art.

As shown in Fig. 4 a top planar view of assembly of embodiment shown in Fig. 3 is shown, with the top and bottom segments of material 22 folded over. The top and bottom segments of material 22 are then conceptually divided into segments 22a and 22b, both at the top and the bottom of the composite, alternating continuously in what will eventually become physically divided portions which will be the front left (top 22aL), front right (bottom 22aR) and the back left (top 22bL) and back right (bottom 22bR) side panel portions.

In areas 22aR and 22aL (front left and right side panel portions), the folded segments of material 22 are temporarily bonded to the underlying (or overlying) layer(s) of stretch material 20. The temporary bonding can be done for instance at spaced apart tack bond sites. The purpose of the temporary bond is to provide control of the material throughout the high-speed manufacturing process, but to allow the bond to become detached when the lap seam is eventually formed, and worn about the waist of a user.

In areas 22bR and 22bL (rear left and right side panel portions), the folded segments of material 22 are ultrasonically or mechanically bonded to the underlying (or overlying) layer(s) of stretch material 20 in zones 26. The bonding can be done in other ways, such as adhesively.

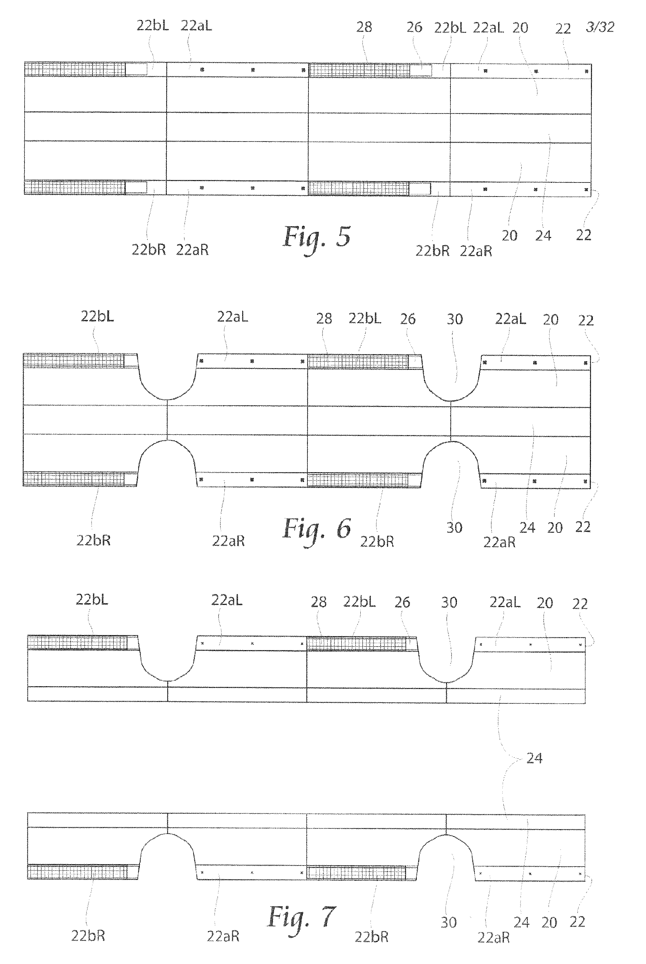

Following this step, and referring now to Fig. 5, after the top and bottom materials 22 have been folded over and bonded in portions to the stretch portions 20 as previously described, a material 28 is applied to the back side panel in areas 22bL and 22bR proximate to and preferably overlying at least in part the bond areas 26 suitable for later attachment to the tack-bonded portions 22. Preferably, the material 28 is a hook type material suitable for attachment to loose loop type material if used for portions 22.

Referring now to Fig. 6, the next step in the process is to die cut areas 30 from side panel assembly which can be discarded or preferably recycled. Preferably, portions 30 of the side panel assembly are removed by die cut or other means to facilitate shaping of the side panel to conform with the waist or leg openings of the diaper configuration, shaped to conform to the body of the wearer.

Next, as shown in Fig. 7, the side panel assemblies are slit, preferably in the middle of the non-stretch panel 24, and spread apart to increase the distance between the left side panels (22aL and 22bL) and the right side panels (22aR and 22aL) panels to form left and right intermediate assemblies. Next, the front panel (22aL and 22aR) assemblies are separated from the 22bL rear panel assemblies (22bL and 22bR) panels, for instance by slitting and slip cutting methods (not shown) for deployment onto a continuous top sheet web 12 in the positions as shown in Fig. 8.

As shown on Fig. 8, the non-stretch panel 24 portions of the side panel assemblies are introduced to the top sheet assembly 12 in overlapping fashion, and bonded thereto in the portions of an overlap between the non-stretch panel 24 and top sheet assembly area identified as overlap area 32.

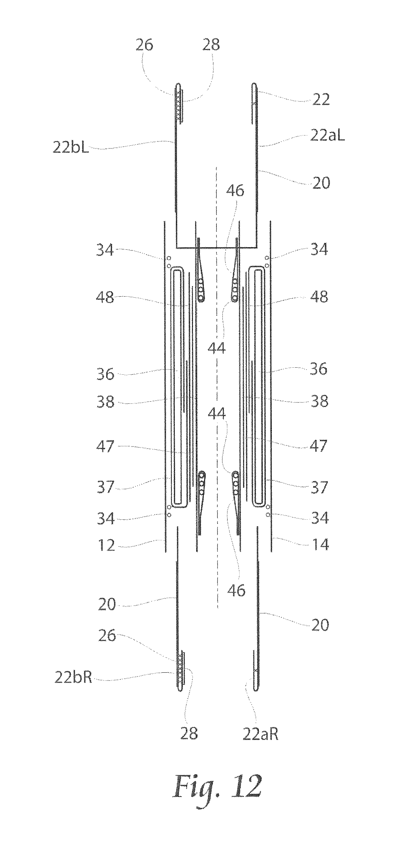

Referring to Figs. 9 and 12 (showing the product cross-section after folding), after slitting the side panel assembly to form independent left (22aL and 22bL) and right (22aR and 22bR) panels, the left panels are further subdivided to form the left front (22aL) and left rear (22bL) panels, and to form the right front (22aR) and right rear (22bR) panels, and introduced to the back sheet material 14. At this point, as shown in Fig. 9, additional components of the diaper panel can be introduced either independently or in pre-constructed fashion. Included are the cuff non-woven 46 containing cuff elastics 44, leg elastics 34, the inner non-woven 38, the absorbent core 36 captured by tissue, the acquisition layer 47, poly layer 40, inner non-woven 38, and waist band 48.

Referring now to Fig. 10, two adjacent products can be separated from one another, for instance, by die cutting, to form independent products 10. Fig. 10 is a top planar view of assembly of embodiment shown in Fig. 9, with the top and bottom non-stretch materials folded over and bonded in portions to the stretch portions, and a hook material applied to the back side panel proximate to the bond area, with portions of the assembly removed by die cut to facilitate shaping of the side panel, and the side panel slit and spread apart, bonded to a top sheet material, and this combination combined with a core and back sheet material, showing the finished product prior to folding.

Referring now to Fig. 11, the diaper product 10 is folded generally at its midsection to form a folded product, with the material 28 being urged against the material 22, to form the refastenable pre-fastened product. In this fashion, the hook material 28 contained on side panel portion 22bL is joined with the side panel portion 22aL, and the hook material 28 contained on side panel portion 22bR is joined with the side panel portion 22aR. This joinder is done by pressure during the folding process, such that the finished product after folding will have pre-sealed sides. If the user desires, the hook material 28 can then be separated from the side panel portions 22aL and 22aR for taking the garment off the user if the product is insulted, or can be opened and re-sealed if the product is still clean.

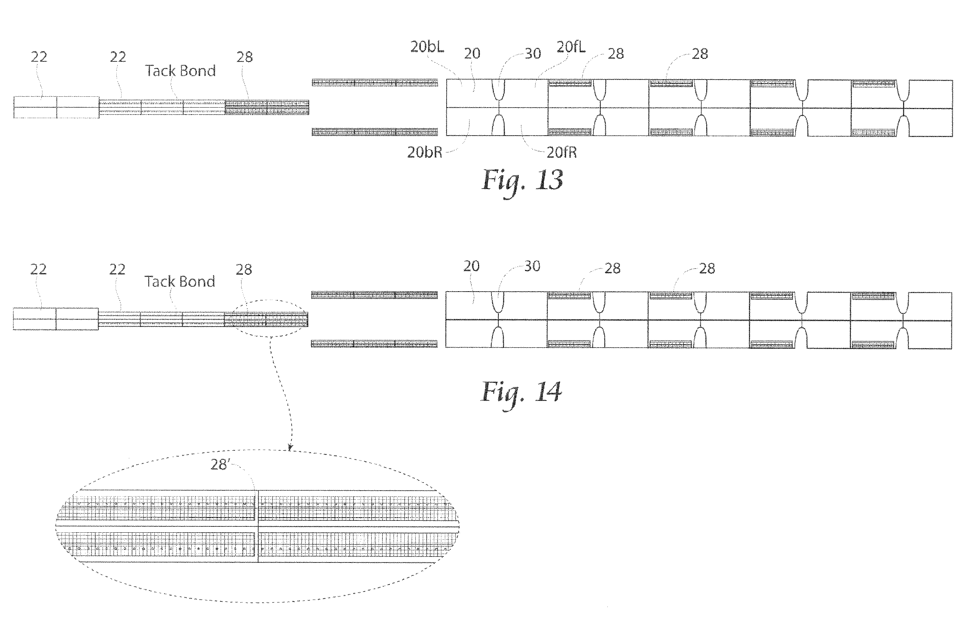

Referring now to Fig. 13, a schematic representation of formation of an alternate embodiment of the present invention is shown, disclosed is a nonwoven tab process with a continuous hook 28 formation, with the hooks 28 away from the body. The formation of this product is much the same as previously described. Working from left to right on Fig. 13, in sequential fashion, non-woven tab material 22 is folded over at the top and the bottom and tack bonded. Next, hook material 28 is coupled to the tack bonded material 22, and material 22 carrying hook material 28 is slit and spread. Next, this composite is introduced to side panel material 20. Die cut areas 30 are removed from the side panel assembly. At this point in the procedure, the construction process resumes at the point previously described with reference to Figs. 6 - 11. To recap, the left and right and front and back panels are slit and separated, as shown in Fig. 7, bonded to the incoming chassis web (Figs. 8, 9), severed into individual diapers (Fig. 10) and folded (Fig. 11) prior to packaging.

Referring now to Fig. 14, a schematic representation of formation of an alternate embodiment of the present invention is shown. In this embodiment, the hook material 28 is applied to tack bonded material 22 discretely (as opposed to continuously, as shown on Fig. 13), with a gap 28' formed between discrete hook portion application. Discrete application of the hook material 28 can be performed, for instanced by slip/cut techniques. In this embodiment, the discrete hook material 28 is not severed following introduction to the web of material 22, instead the combination tab 20/hook 28 material is severed in between hook 28 portions.

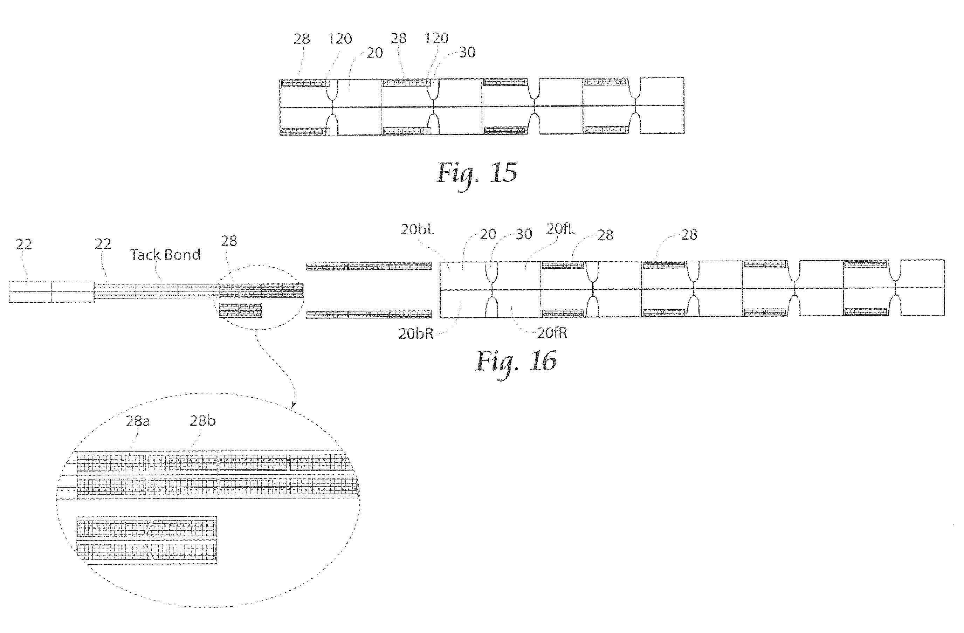

Referring now to Fig. 15, an alternate embodiment of the present invention is shown, in which a nonwoven tab created of material 120 (longer than the hook material 28 segments) is coupled to hook material segments 28, and the material 120 extends into the die cut region 30 such that in use the material 120 would extend nearly the length of the back side panel 20.

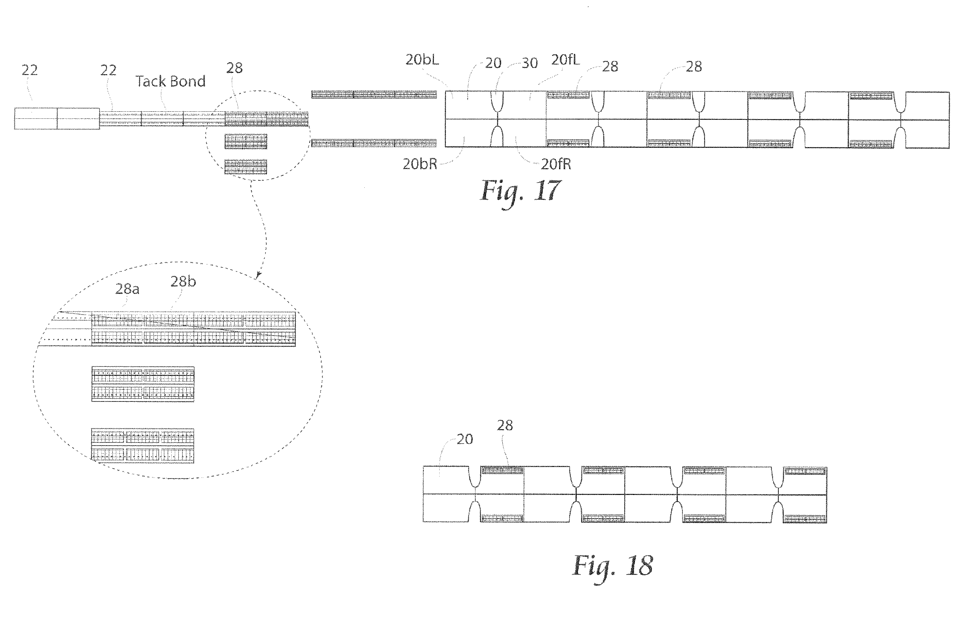

Referring now to Fig. 16, an alternate embodiment is shown, with a discrete hook 28 overlapping the die-cut formation, with the hooks away from the body. In this embodiment, the hook material 28 is introduced such that the hook material 28 overlaps the space where the material is cut prior to introduction onto the side panel. The result is that the hook material 28 resides on the back side panel in two discrete pieces 28a and 28b. The same result is achieved if, as shown in Fig. 17, multiple discrete hook portions 28 are applied to the nonwoven tab 20 material, slit and separated prior to introduction onto the side panels 22.

Referring now to Fig. 18, an alternate embodiment of the present invention is shown with nonwoven tab process with hooks 28 on the front side panel, with the hooks 28 facing away from the body.

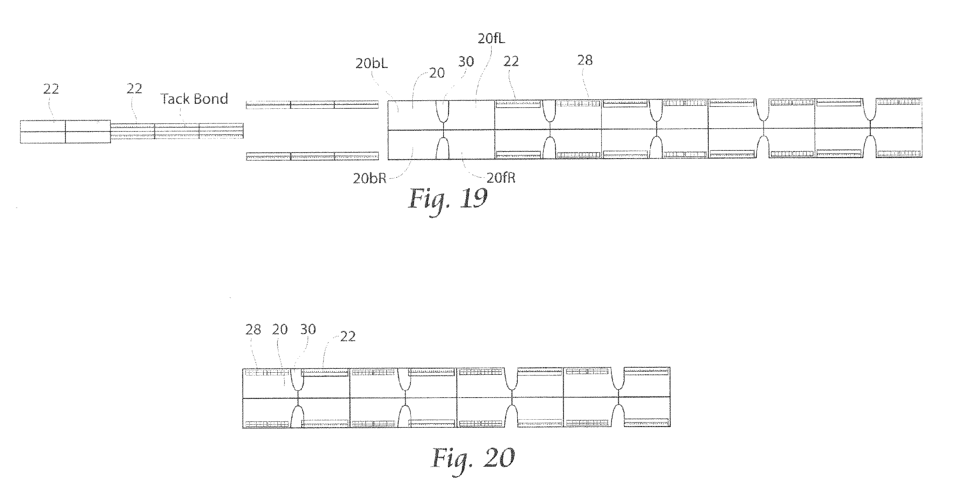

Referring now to Fig. 19 an alternate embodiment of the present invention is shown, with hooks 28 on the front side panel, and the folded over nonwoven 22 on the back panel, with hooks 28 toward the body. Fig. 20 is similar, except that the folded over nonwoven 22 is on the front panel, and the hooks 28 are on the back panel. In the embodiments of Fig. 19 and 20, a nonwoven tab 22 is first folded over itself, tack bonded, slit and spread, and then applied intermittently to either the back panel of web 20 (Fig. 19) or the front panel of web 20 (Fig. 20), in addition to the hook material 29 being applied to either the front panel of the web 20 (Fig. 19) or the back panel of web 20 (Fig. 20).

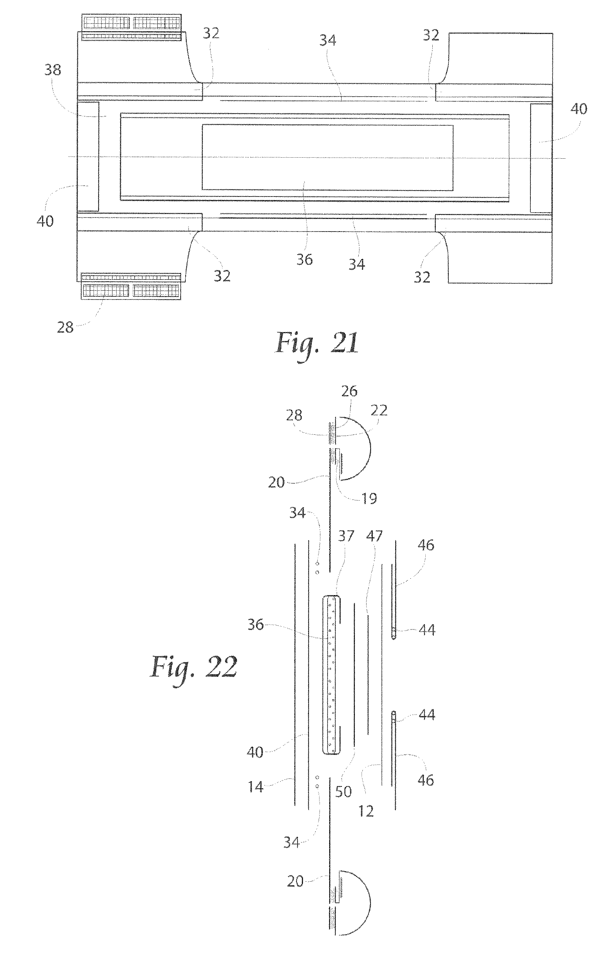

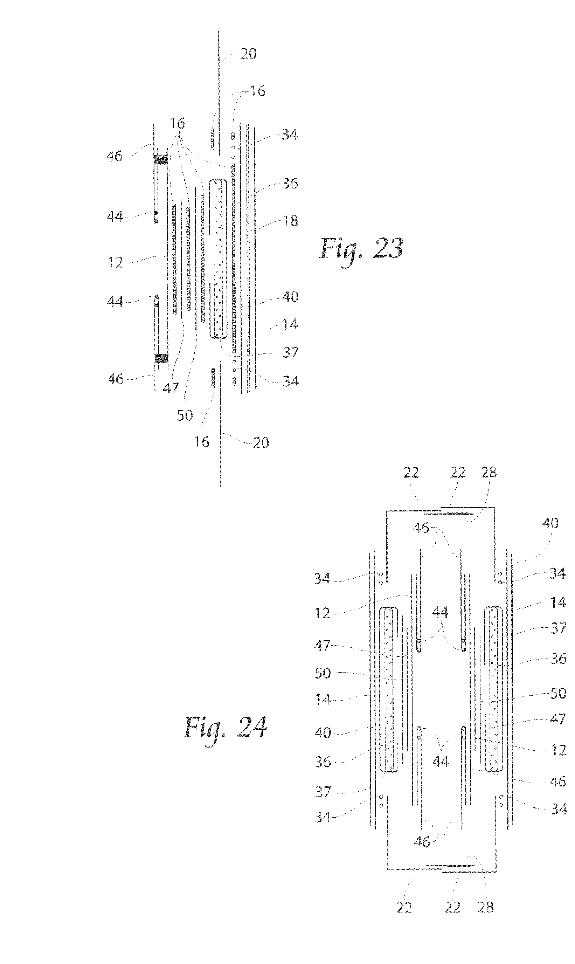

Referring now to Fig. 21, a plan view of a product produced according to the present invention is shown, with a back side panel cross section shown in Fig. 22, a front side panel construction shown in Fig. 23, and a lap-seam full product cross section shown in Fig. 24. Referring to Fig. 22 showing the back panel cross section, undergarment 10 is formed with top sheet 12, back sheet 14, bonded where desired with adhesive 16 or ultrasonic bonds 18, or tack bonds 19. A poly layer 40 is provided, as are leg elastics 34, panels 20, absorbent core 36 carried by core wrap 37 (preferably non-woven), top tissue 50, acquisition layer 47, and cuff elastics 44 carried by cuff non-woven 46.

Referring to Fig. 23 showing the front panel cross section, the top sheet 12 is coupled to the cuff 46, and the acquisition layer 47, the top tissue 50, and next the front panels about the core 36 carried by the core wrap 37. Leg elastics 34 are provided, and completing the cross section is poly 40 coupled to the back sheet 14, and adhesives 16 are applied where necessary for bonding.

Fig. 24 shows the lap seam (overlap) provided by the hook material 38, releasably coupled about the waist of a user with material 22.

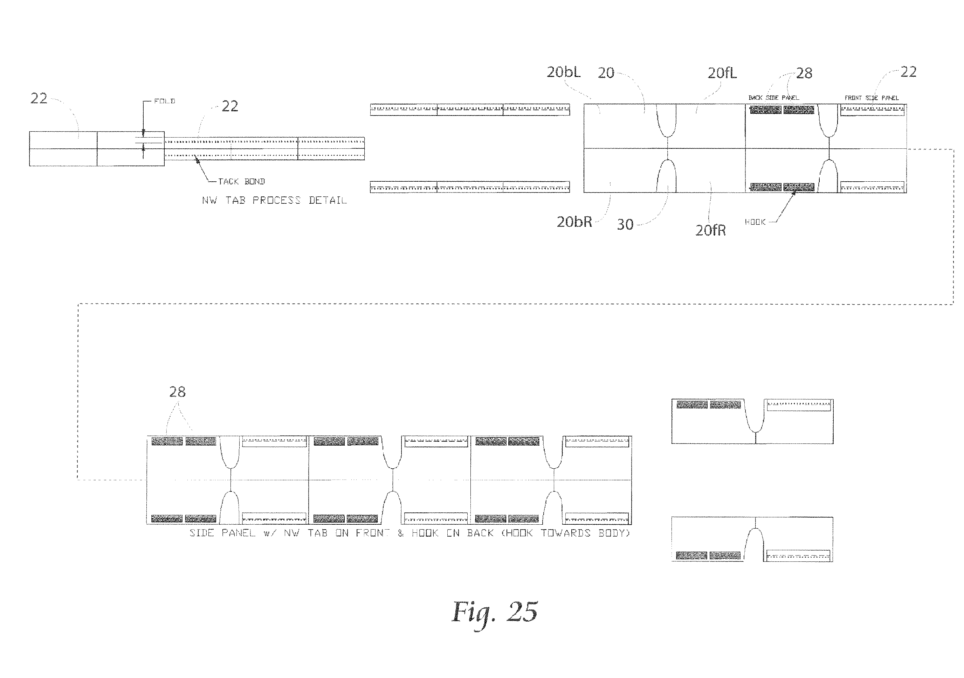

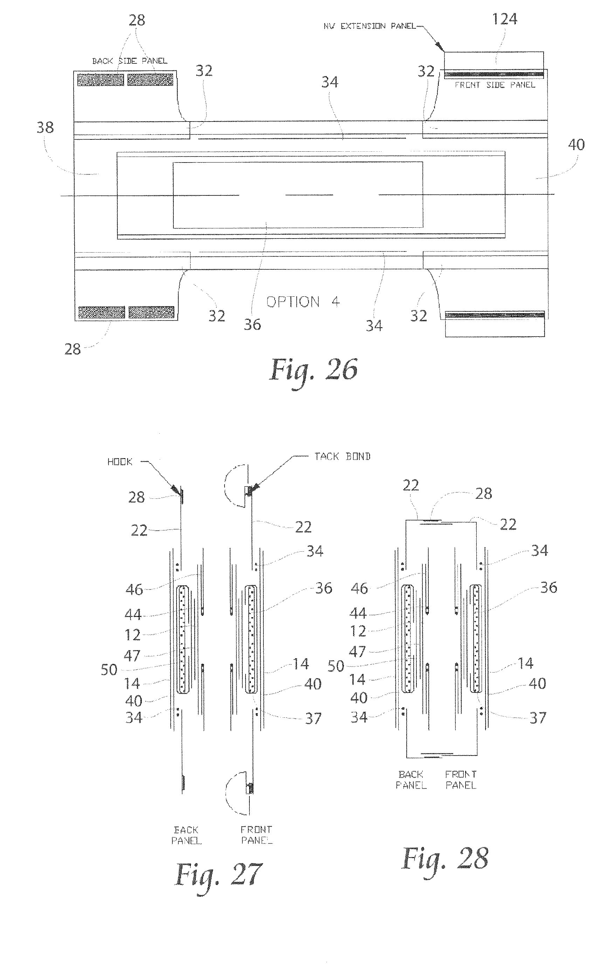

Referring now to Figs. 25-28, in-process, plan, and side views of a fourth alternate embodiment of a diaper of the present invention are shown. In this embodiment, side panel 20 carries a nonwoven tab portion 22 on front panels 20fL and 20fR and a hook material 28 on the back panels 20bL and 20BR, and nonwoven extension panel 125 shown in a laid out position, previously attached to the front panels 20fL and 20fR as folded over material 22.

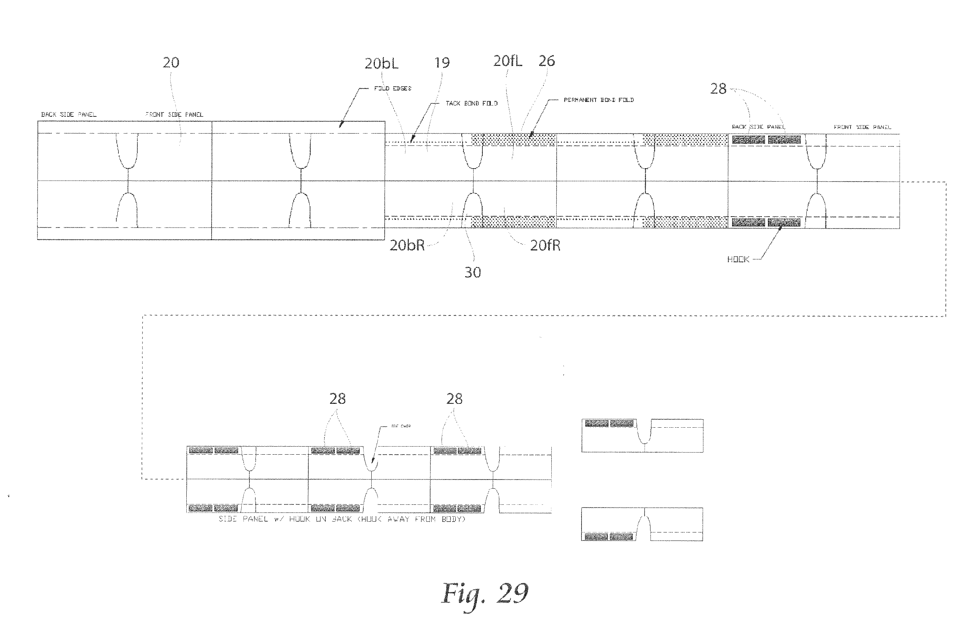

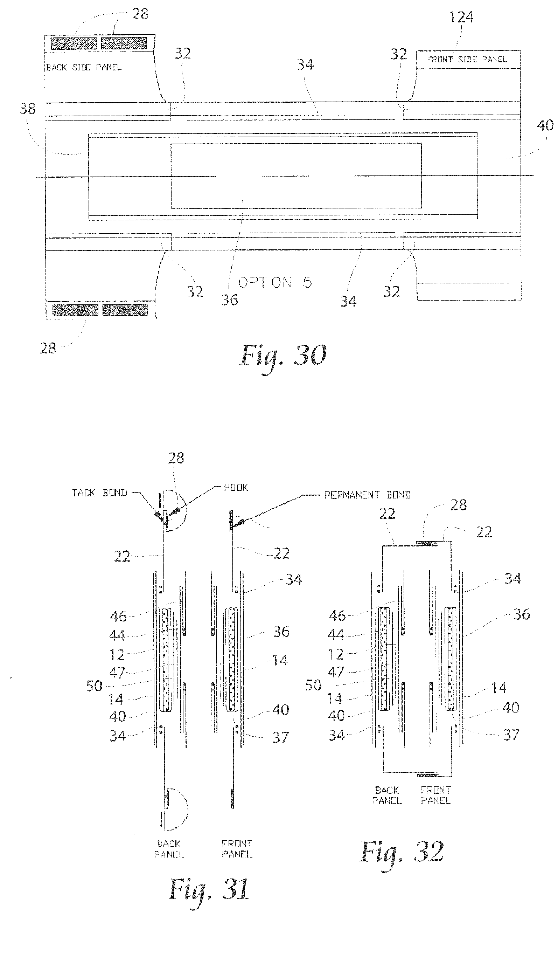

Referring now to Figs. 29-32, in-process, plan, and side views of a fifth alternate embodiment of a diaper 10 of the present invention are shown. In this embodiment, a larger side panel web 20 is used in place of the non-woven tab 22 process shown in Figs. 25-28. Hook material 28 is on back panels 20bL and 20BR and facing away from the body when worn. In this embodiment, a portion of the side panel web 20 is folded over at the top and bottom of the web 20. A permanent bonding zone 26 is established on front panels 20fL and 20fR at the folded over portion of web 20, and tack bonds 19 couple back panels 20bL and 20BR to the folded over portion of web 20. Hook material 28 is applied, and the web is die cut, slit and spread as described previously.

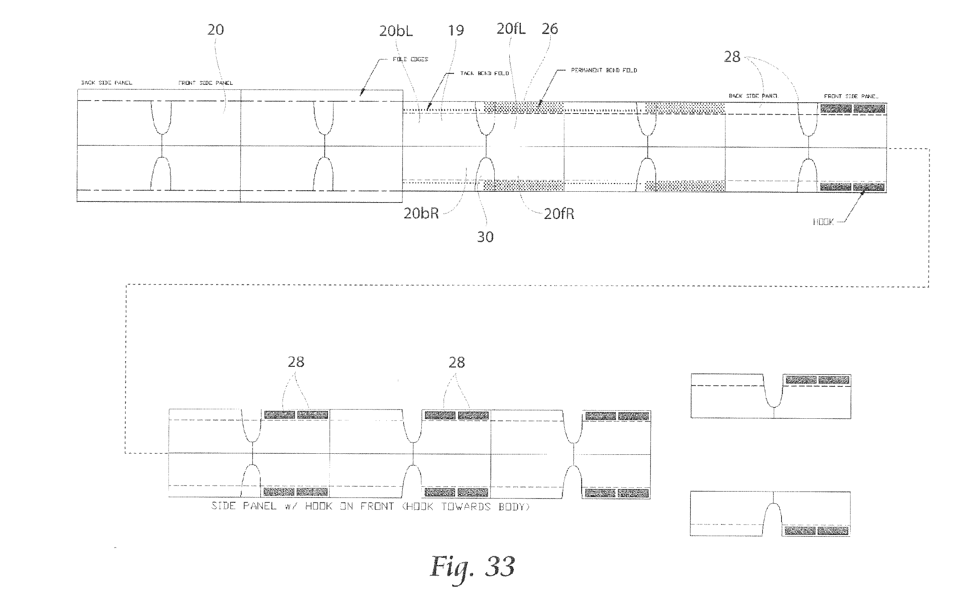

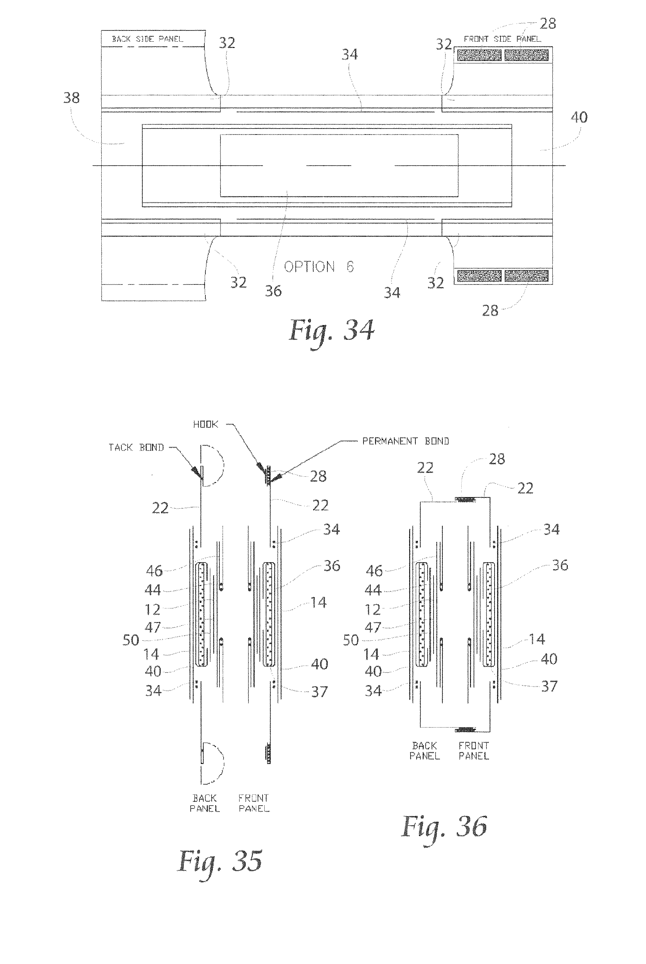

Figs. 33-36 shown a sixth alternate embodiment of a diaper of the present invention, similar to the embodiment of Figs. 29-32, except that the hook material 28 is on front panels 20fL and 20fR and facing towards the body when worn.

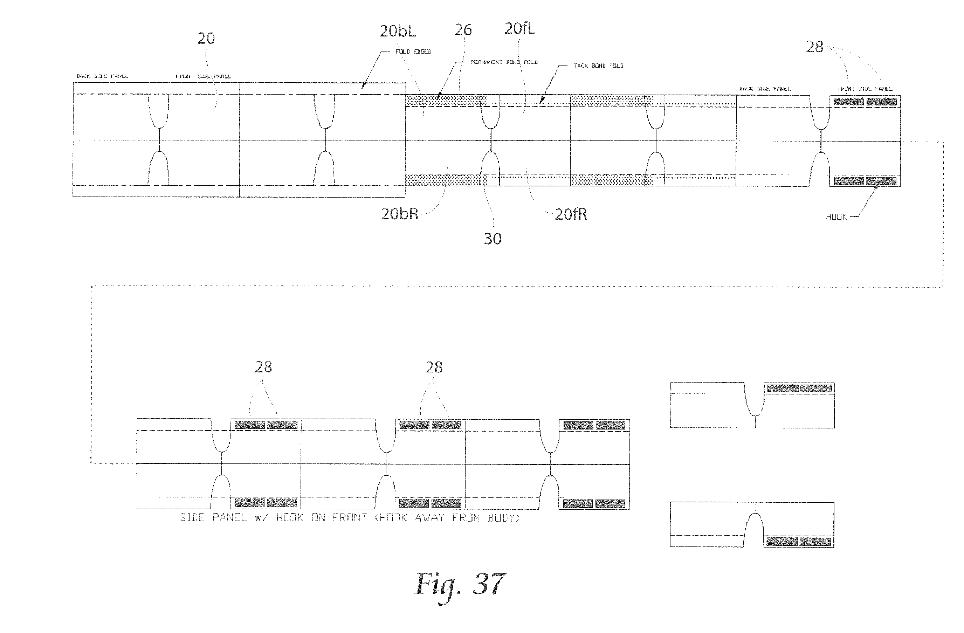

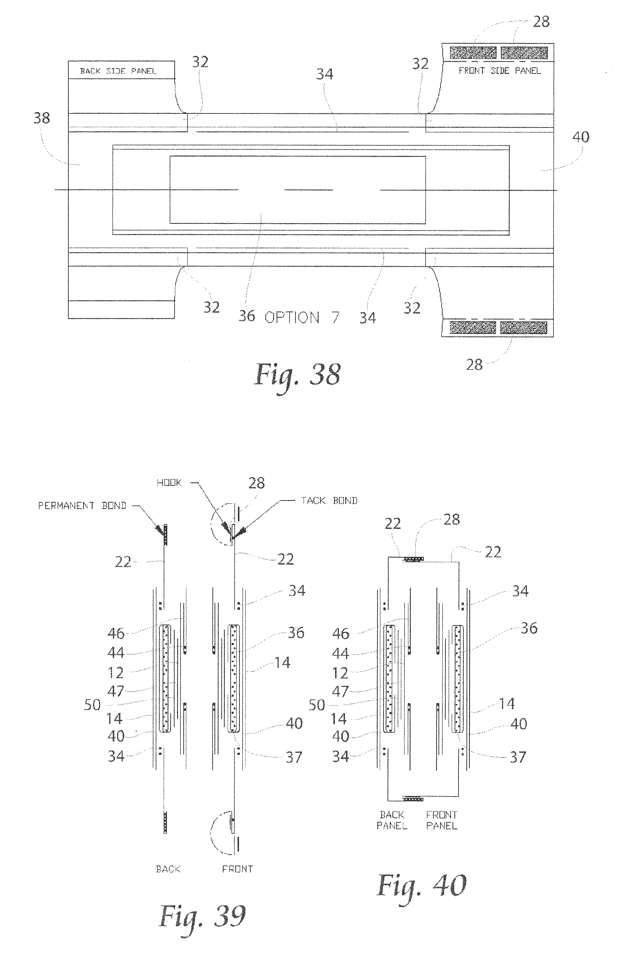

Referring now to Figs. 37-40 in-process, plan, and side views of a seventh alternate embodiment of a diaper of the present invention are shown, similar to the embodiment of Figs 33-36. In this embodiment, permanent bonding zone 26 is established on back panels 20bL and 20BR at the folded over portion of web 20, and tack bonds 19 couple front panels 20fL and 20fR to the folded over portion of web 20. The hook material 28 is applied to front panels 20fL and 20fR, with hooks 28 facing away from the body.

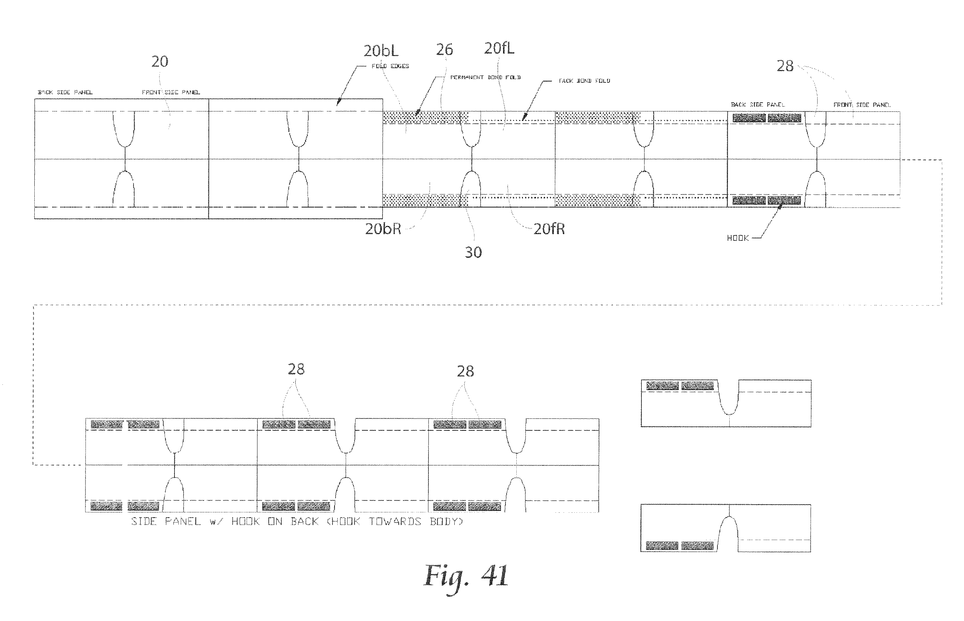

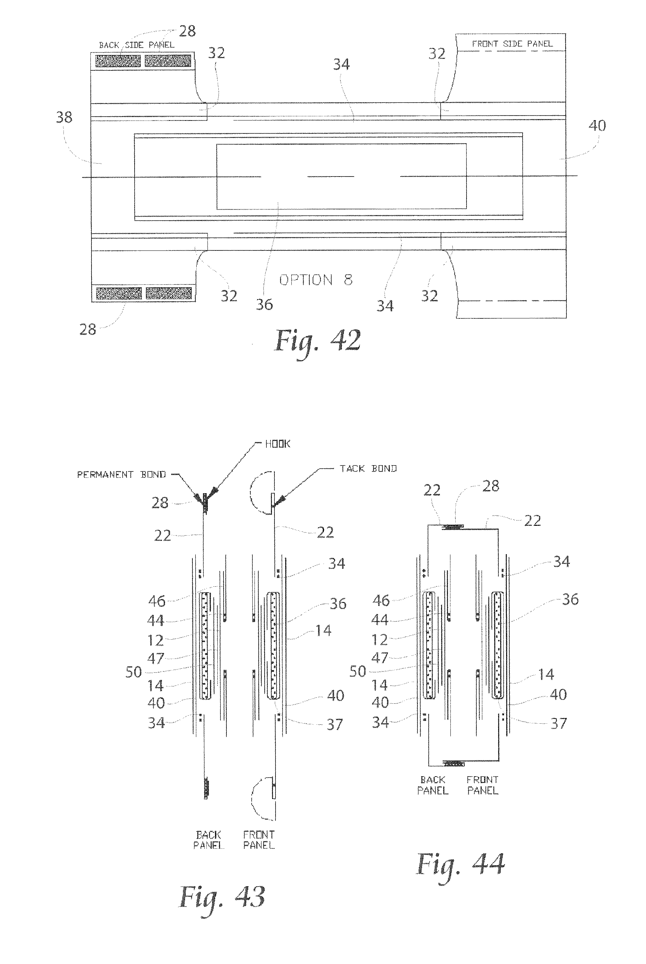

Referring now to Figs. 41-44 in-process, plan, and side views of an eighth alternate embodiment of a diaper of the present invention are shown, similar to the embodiment of Figs 37-40. In this embodiment, the hook material 28 is applied to back panels 20bL and 20bR, with hooks 28 facing towards the body.

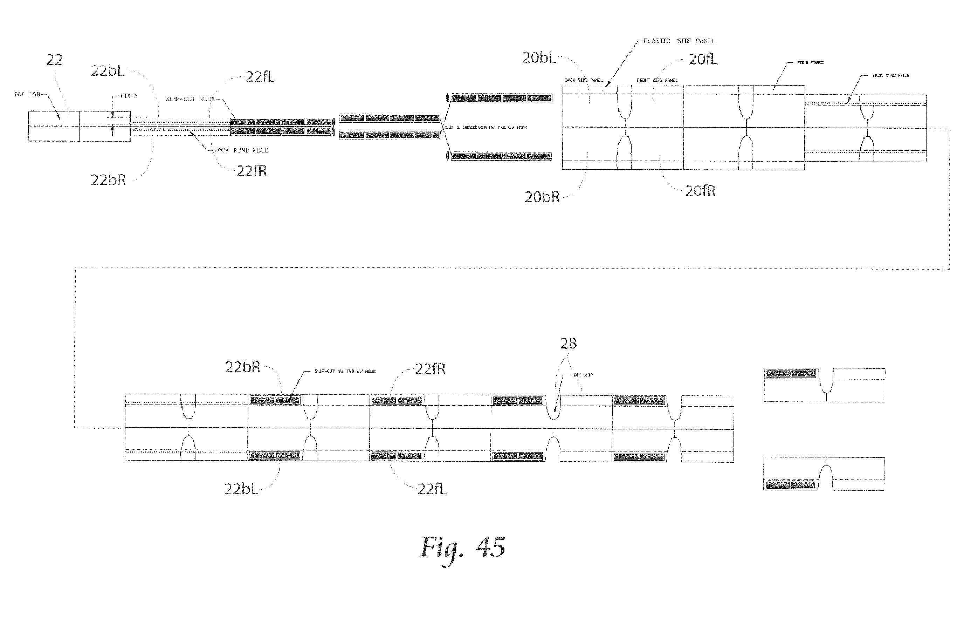

Referring now generally to Figs. 45-64, several variations of manufacturing techniques and diaper products produced thereby are shown. In general, these options are for putting on an extension panel attached to the outside surfaces of elastic front and back side panels. This extension panel (for instance, formed of a non-woven material) will be coupled to at least one of a back or front panel. The hook portion may or may not be attached to the nonwoven extension panel, and the hook may face the body or away from the body. The nonwoven extension panels wind up on the outside of the product in all four of the attached options. The end result is a refastenable lap seam.

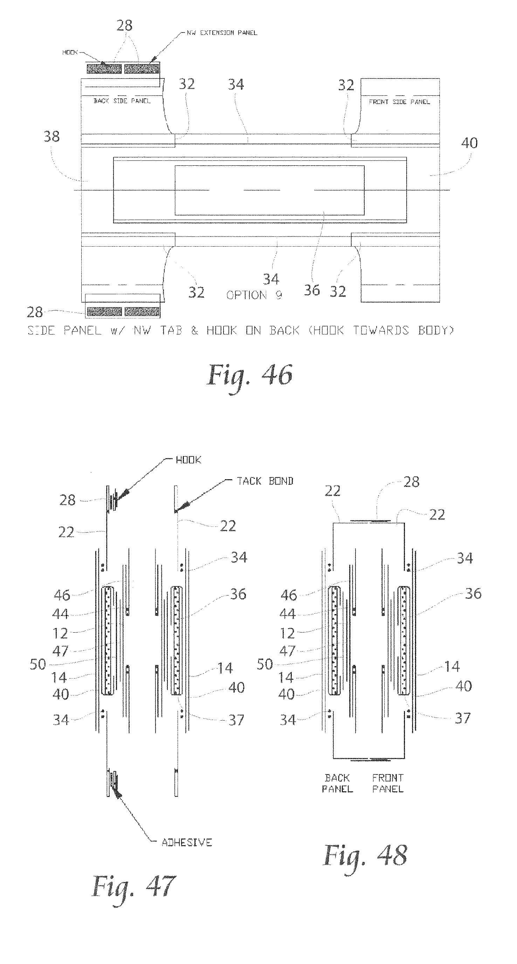

Referring now to Figs. 45-48 in-process, plan, and side views of a ninth alternate embodiment of a diaper of the present invention are shown. In this embodiment, the non-woven tab material 22 is folded over and tack bonded to itself. Hooks 28 are slip/cut onto the folded over portions of non-woven 28 along back left portion 22bL, front left portion 22fL, back right portion 22bR and front right portion 22fR of web 22. The back left portion 22bL, front left portion 22fL, back right portion 22bR and front right portion 22fR of web 22, all carrying hook material 28, segmented where desired, are slit and crossed over such that the back left portion 22bL and front left portion 22fL of web are actually coupled to back right portion and front right portions 20bR and 20fR, and the back right portion 22bR and front right portion 22fR of web are actually coupled to back left portion and front left portions 20bL and 20fL, after the larger side panel web 20 is folded over itself along the top and bottom edges and tack bonded to itself. The back left portion 22bL, front left portion 22fL, back right portion 22bR and front right portion 22fR of web 22 are slip/cut onto the folded over portions of the panel web 20 as described. The panel web 20 is then slit and spread, and placed as described with reference to Figs. 7-8. The result is a side panel with nonwoven tab 22 and hook 28 on the back 20bl and 20bR, with the hook 28 towards the body, as shown in plan and cross section in Figs. 46-48.

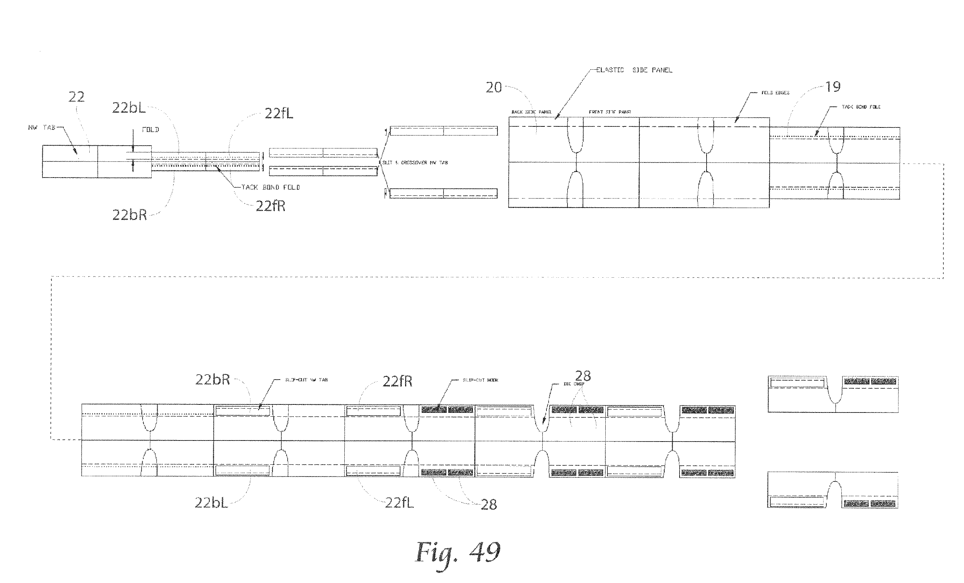

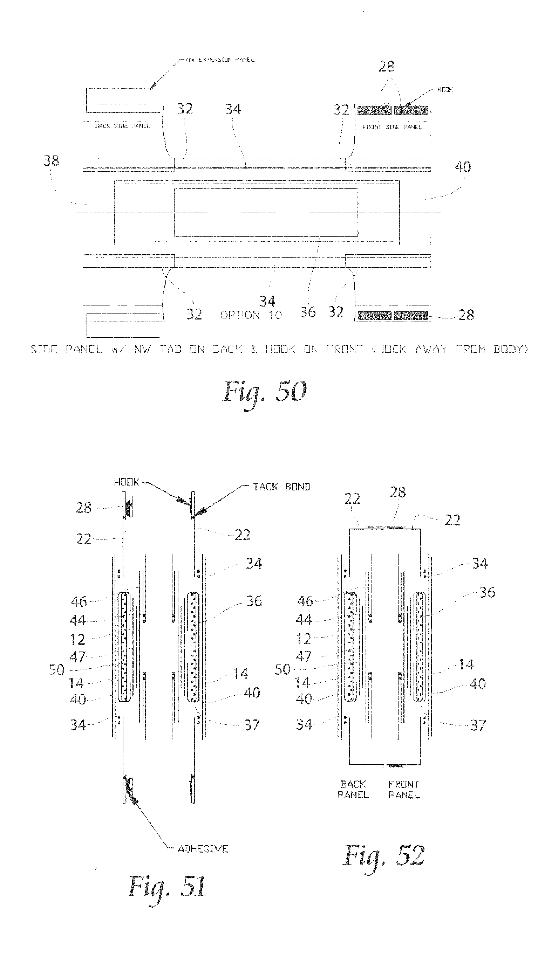

Referring now to Figs. 49-52 in-process, plan, and side views of a tenth alternate embodiment of a diaper 10 of the present invention are shown. In this embodiment, the non-woven tab material 22 is folded over and tack bonded to itself, forming tabs 22bL, 22fL, 22bR and 22fR. These segments are slit and crossed over, and slip cut onto web 20, itself having been previously folded over along its edges and tack bonds 19 applied. Segments 22bL, 22fL, 22bR and 22fR are slip/cut into back panel positions 20bL and 20bR, as shown, and hook segments 28 are slip cut onto front panel positions 20fL and 20fR. The panel web 20 is then slit and spread, and placed as described with reference to Figs. 7-8. The result is a side panel with non-woven tabs 22 on back panel portions 20bL and 20bR, and hook material 28 on front panel positions 20fL and 20fR, with hooks 28 facing away from the body, as shown in plan and cross-section in Figs. 50-52.

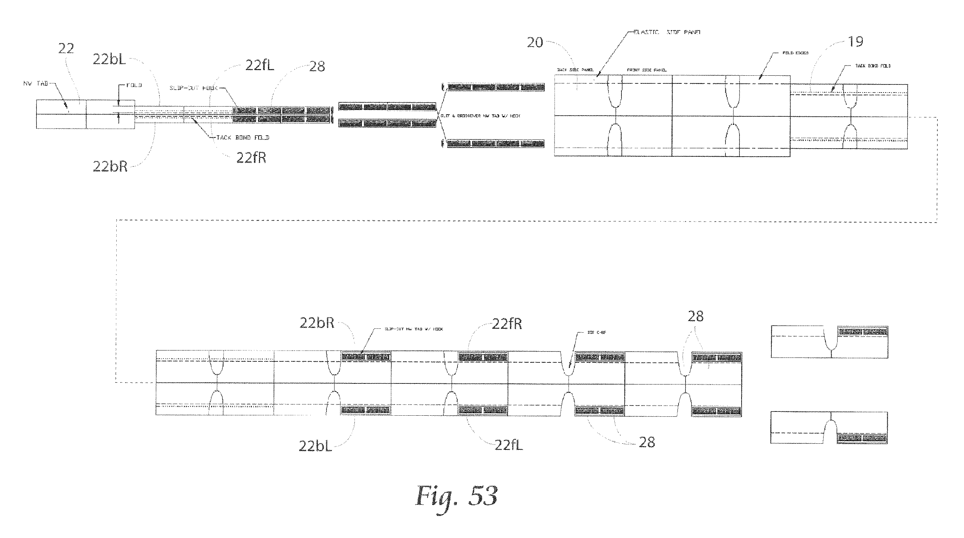

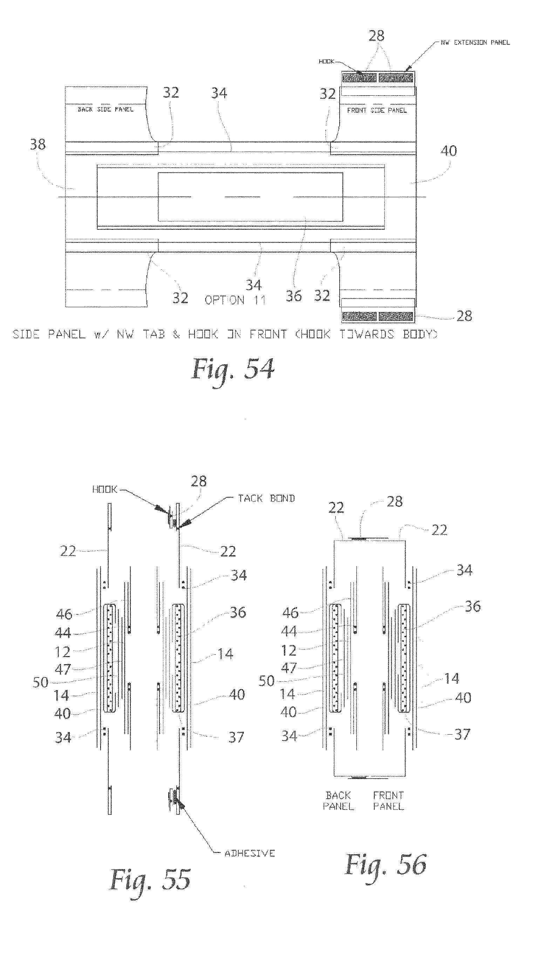

Referring now to Figs. 53-56 in-process, plan, and side views of an eleventh alternate embodiment of a diaper of the present invention are shown. This embodiment is similar to the embodiment shown in Fig. 49, with the exception that hook material 28 is slip cut onto portions 22bL, 22fL, 22bR, and 22fR prior to those portions being slit, and crossed over and placed onto portions 20fL and 20fR. The result is a side panel with nonwoven tab 22 and hook material on the front panels 20fL and 20fR, with hook material 28 facing towards the body, as shown in plan and cross section in Figs. 54-56.

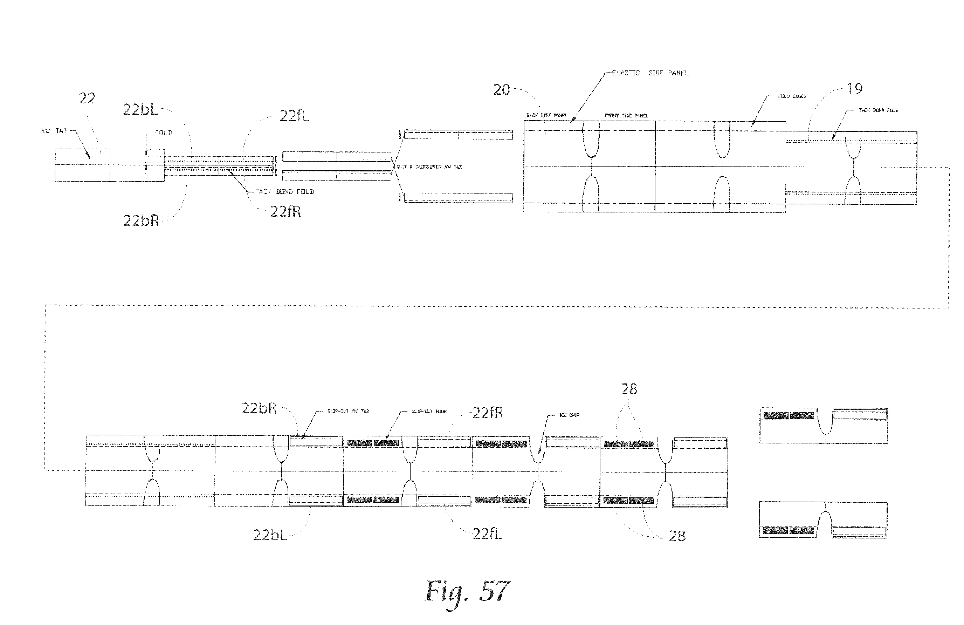

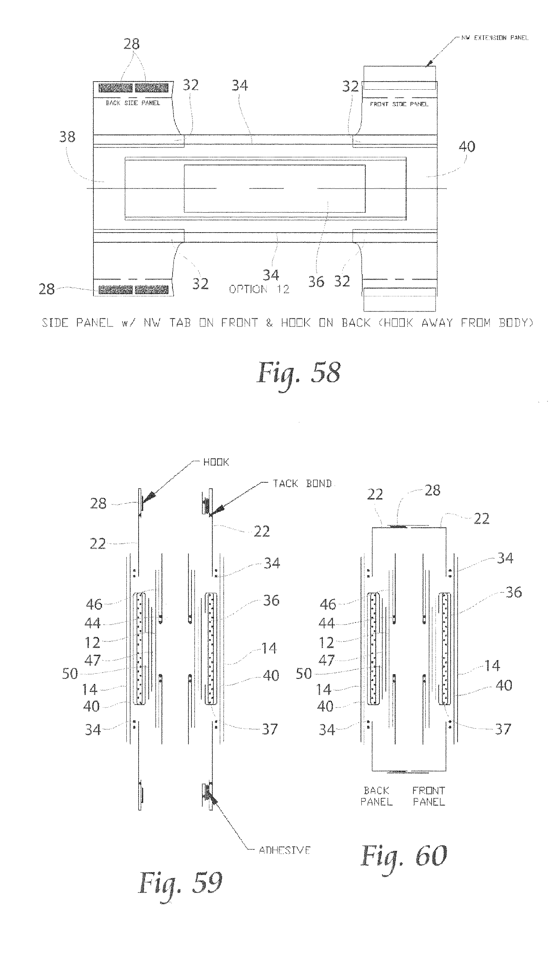

Referring now to Figs. 57-60 in-process, plan, and side views of a twelfth alternate embodiment of a diaper 10 of the present invention are shown. In this embodiment, the non-woven tab material 22 is folded over and tack bonded 19 to itself. Portions 22bL, 22fL, 22bR, and 22fR are slit and crossed over for application to portions 20fR and 20fL over the elastic side panel 20, which has been previously folded over and tack bonded to itself. Hooks 28 are slip/cut onto back side panel portions 20bL and 20bR. The panel web 20 is then slit and spread, and placed as described with reference to Figs. 7-8. The result is a side panel with nonwoven tabs 22 on front side panel portions 20fL and 20fR, hook material 28 on the back side panel portions 20bL and 20bR, with hook material facing away from the body, as shown in plan and cross section in Figs. 58-60.

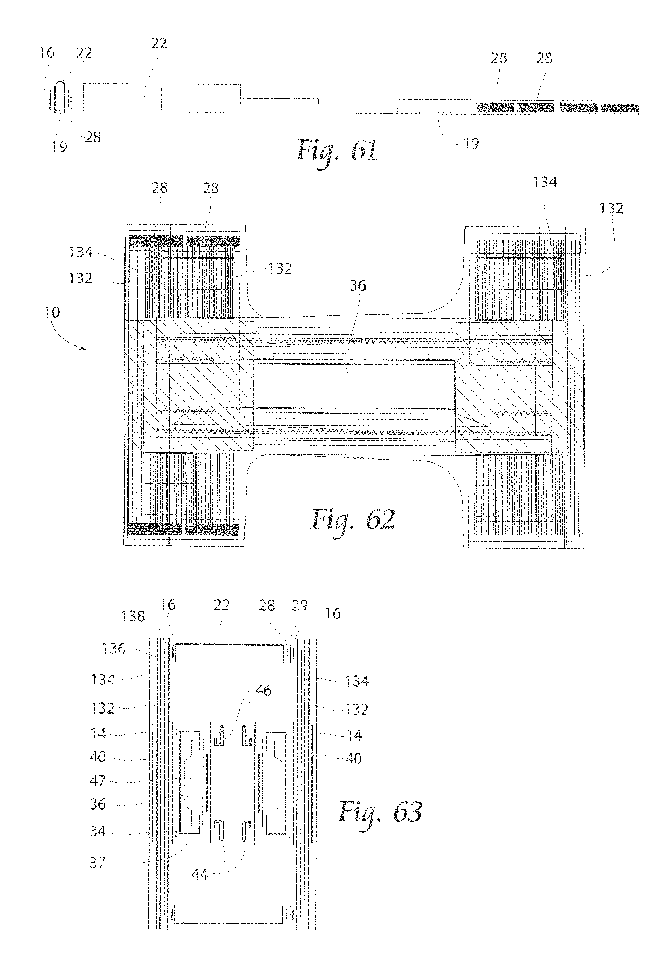

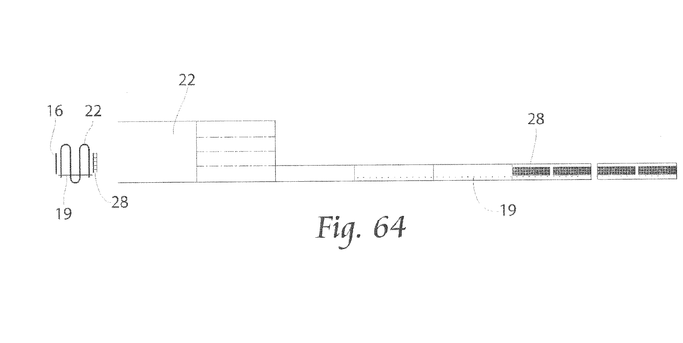

Referring now to Figs. 61-63, a thirteenth embodiment of diaper 10 is shown. In this embodiment, two hinges are added to the side seam areas which are created in offline unwinds of material 22 and slip/cut to the inner nonwoven front panel 20. Nonwoven side panel portions 22 are folded in half (or, in the case of Fig. 64 for a fourteenth embodiment, folded in fourths), and tack bonds 19 created to bond the material 22 to itself. Next, hook material 28 is slip/cut onto the folded over non-woven portions 22, and adhesive 16 is applied to the folded over 22 generally on a portion where hook material 28 is not present. The addition of loop 29 (see Fig. 63) to the opposite side of the product may also be desired if hook material 28 does not stick to the nonwoven panel 20 material, depending on user preference in choice of material 20. This embodiment refastens on the inside of the product 10. Next, the composite web 16/22/19/28 is then slit and spread, and placed as described with reference to Figs. 7-8 onto side panel web 22 as previously described. As shown in Fig. 63, this embodiment can also include waist band elastics 132, side panel elastics 134, and optional core nonwoven layers 136 and 138.

The foregoing is considered as illustrative only of the principles of the invention. Furthermore, since numerous modifications and changes will readily occur to those skilled in the art, it is not desired to limit the invention to the exact construction and operation shown and described. While the preferred embodiments have been described, the details may be changed without departing from the invention, which is defined by the claims.

## Claims

1. A disposable product comprising:
a front left side panel folded over onto itself;
a front right side panel folded over onto itself;
a back left side panel folded over onto itself;
a back right side panel folded over onto itself
a tab material folded over onto itself;
said tab material carried by at least one of the pair of said front left side panel and said front right side panel folded over onto itself, and the pair of said back left side panel and said back right side panel;
a hook material carried by at least one of said tab material and said at least one of the pair of said front left side panel and said front right side panel folded over onto itself, and the pair of said back left side panel and said back right side panel.

2. A disposable product according to claim 1, said front side panels carrying said tab material, said back side panels carrying said hook material, said hook material in a body facing orientation when worn.

3. A disposable product according to claim 1, said back side panels carrying said hook material, said hook material in a body away facing orientation when worn.

4. A disposable product according to claim 1, said back side panels carrying said hook material and said tab materials to form an extension panel, said hook material in a body facing orientation when worn.

5. A disposable product according to claim 1, said back side panels carrying said tab material, said front side panels carrying said hook material in a body away facing orientation when worn.

6. A disposable product according to claim 1, said front side panels carrying said hook material and said tab materials to form an extension panel, said hook material in a body facing orientation when worn.

7. A disposable product according to claim 1, said tab material carrying said hooks on a first folded over face, and carrying an adhesive on a second folded over face.

8. A disposable product according to claim 1, said tab material forming an extension panel, said front side panels carrying said hook material.

9. A disposable product according to claim 1, said tab material forming an extension panel carrying hook material.

10. A disposable product comprising:
a front left side panel folded over onto itself;
a front right side panel folded over onto itself;
a back left side panel folded over onto itself;
a back right side panel folded over onto itself
a hook material carried by at least one of said tab material and said at least one of the pair of said front left side panel and said front right side panel folded over onto itself, and the pair of said back left side panel and said back right side panel.
